# EUROPEAN PATENT SPECIFICATION

(11) **EP 1 814 387 B1**
(45) Date of publication and mention of the grant of the patent: **09.12.2009**
(21) Application number: 05813221.8
(22) Date of filing: 17.11.2005
(51) Int. Cl.: A01K 67/027, G01N 33/00, C12N 15/85

(54) **ANIMAL MODEL FOR STUDYING SUBSTANCES WITH CARDIOTHERAPEUTIC AND ANTI-HYPERTENSIVE ACTIVITY**
TIERMODEL FÜR DIE UNTERSUCHUNG VON SUBSTANZEN MIT HERZTHERAPEUTISCHER UND BLUTDRUCKSENKENDER WIRKUNG
MODELES ANIMAUX POUR L'ETUDE DE SUBSTANCES A ACTIVITE CARDIOTHERAPEUTIQUE ET HYPERTENSIVE

(30) Priority: 18.11.2004 IT RM2004 569
(43) Date of publication of application: 08.08.2007
(73) Proprietor: Biogem s.c.a.r.l, 83031 Ariano Irpino (AV) (IT)
(72) Inventor: POTENZA, Nicoletta, c/o Biogem s.c.a.r.l., 83031 Ariano Irpino (AV) (IT); DI LAURO, Roberto, c/o Biogem s.c.a.r.l., 83031 Ariano Irpino (AV) (IT); DE VITA, Gabrielle, c/o Biogem s.c.a.r.l., 83031 Ariano Irpino (AV) (IT); LEMBO, Giuseppe, c/o Biogem s.c.a.r.l., 83031 Ariano Irpino (AV) (IT)
(74) Representative: Capasso, Olga
(86) International application number: PCT/IT2005/000668
(87) International publication number: WO 2006/054326

(56) References cited:
- WO-A-94/11506
- KOERA K. ET AL.: "K-ras is essential for the development of the mouse embryo" ONCOGENE, vol. 15, 1997, pages 1151-1159, XP002376421 cited in the application
- ESTEBAN L.M. ET AL.: "Targeted disruption of H-ras and N-ras, individually or in combination, reveals the dispensability of both loci for mouse growth and development." MOL. CELL BIOL., vol. 21, no. 5, March 2001 (2001-03), pages 1444-1452, XP002376422 cited in the application
- POTENZA N. ET AL.: "Replacement of K-Ras with H-Ras supports normal embryonic development despite inducing cardiovascular pathology in adult mice." EMBO REPORTS, vol. 6, no. 5, 29 April 2005 (2005-04-29), pages 432-437, XP002376423

## Description

### Introduction

The three mammal ras genes, H-, N- and K-ras encode for proteins of 21 kDa (H-Ras, N-Ras, and 2 alternative splicing products of the K-ras gene, K-ras 4A and K-ras 4B, the predominant isoform), members of the superfamily of monomeric GTPases. These proteins are crucial molecules for signal transduction: they are located on the inner surface of the plasmatic membrane where they receive different extracellular signals, originated by membrane receptors, able to induce the reversible transformation from an inactive conformation, bound to the GDP, to an active conformation, bound to the GTP. Through this regulated cycle between the active state and the inactive state, the Ras proteins act as molecular switches in the transduction of extracellular stimuli, regulating proliferation, differentiation and apoptosis¹⁻³.

Ras genes are among the most frequently mutated in human tumours (up to 35%) and animal tumours. Mutations cause Ras proteins to be constitutively bonded to GTP, hence in the active conformation, thus giving an uncontrolled induction signal⁴_{.}

The three mammal ras genes encode very similar products that differ almost exclusively in the carboxy-terminal portion, and share, in the amino-terminal portion, a highly homologous domain. The other degree of identity and the same capability, by the three mutated genes, to cause transformation in different cell types, suggest that the four homologous proteins are functionally interchangeable. However, it is not yet clear whether the Ras isoforms also have individual roles.

Different observations suggest that Ras proteins have common functions and are functionally redundant. In fact: (i) the three ras genes are expressed simultaneously in many murine and human tissues⁵; (ii) in many tumour types, there is no absolute specificity for a particular mutated ras gene; (iii) the four Ras proteins have a sequence identity for the first 86 aminoacids, i.e. the portion including the domain responsible for interaction with the effectors (aa. 25-45), suggesting an identical ability to interact with them; (iv) in mice, the function of the Ras genes is in part not essential for development, growth and fertility. N-ras and H-ras knock-out mice, both individually and in combination, are viable and show no abnormality, both during development and after birth⁶⁻⁸. Knock-out mice for the minor isoform of K-ras, K-ras 4A, are also healthy and fertile⁹.

On the other hand, there is also evidence that each gene of the Ras family has a specific role. For example: (i) although the expression of Ras is ubiquitous, the levels of Ras mRNA in mice seem to be regulated both temporally and spatially:
tissue-specific and development stage-specific quantitative differences of the levels of expression of the mRNA of each of the Ras genes have been described; K-ras and N-ras have similar expression profiles^{5, 10-11}; (ii) some tumour types are associated with the mutation of a particular Ras gene, suggesting a specific oncogenic role for each of these genes in some tissues¹: H-Ras mutations are preferentially found in skin tumours and in squamous carcinomas of the neck and of the head; K-ras mutations are mainly found in lung tumours (about 25-50%), colon tumours (about 50%) and pancreas tumours (aabout 70-90%); N-Ras mutations are common in hematopoietic tumours; (iii) Ras proteins show a great sequence divergence in the last 25 aminoacids in the C-terminal region, which contains different signals that mediate different post-transductional modifications. It is demonstrated that different post-transductional modifications of the four Ras proteins cause a different localisation on the plasmatic membrane and, consequently, the recognition of different subgroups of effectors, thus leading to different biological effects¹²⁻¹³; (iv) in contrast with the fact that knock-out mice for H- and N-ras are viable, K-ras knock-out mouse embryos die between day 12 and the end of gestation, due to defects in the foetal liver, anaemia, defects in the myocardium cell proliferation and increased cell death of the motoneurons of the ventral horns of the elongated caudal marrow and of the cervical spinal marrow¹⁴⁻¹⁵.

The embryo lethality observed for K-ras knock-out mice, but not for H-ras or N-ras knock-out mice, could be explained with two different hypotheses: 1) K-ras has a unique, essential function in development, because it activates a specific mechanism of signal transduction, not shared with the other Ras proteins, necessary and sufficient for the development of the mouse; 2) the need for the presence of K-ras during development is due to its predominant or exclusive expression in specific compartments, or in particular development stages. These possibilities are both valid in view of current knowledge.

The authors of the present invention have produced an experimental model of mouse by knocking-in an encoding H-ras sequence into K-ras locus. In these mice, both copies of the K-ras gene contain the encoding region of H-ras under the control of the regulatory regions of the K-ras gene (K-ras ^{(Hras)}, hereafter called HrasKI). The rearranged locus was designed in such a way as to exclude the possibility that any genic product of K-ras may be synthesised. The mice are viable and H-rasKI embryos exhibit none of the defects observed in K-ras knock-out embryos. These results demonstrate that K-Ras is not necessary for the development of the mouse, since H-Ras is able to replace K-Ras during development.

However, the authors have found that HrasKI adult mice exhibit a considerable increase in systolic and diastolic arterial pressure, whereto is associated with the emergence of a dilated cardiomyopathy. These pathologies are not observed in the murine model used as experimental control having the encoding region of K-ras 4B isoform knocked-in in its same locus (K-ras ^{(Kras)}), hereafter called KrasKI. These animals were produced using the same strategy as to produce the H-rasKI mice.

Therefore, the authors' results assign to K-ras a new biological function in the control of cardiovascular physiology.

The emergence of myocardium hypertrophy, which evolves to dilated cardiomyopathy and heart failure is a pathological sequence that occurs often in the natural history of arterial hypertension in humans, and it is a major cause of cardiovascular morbidity and mortality ¹⁶⁻¹⁸. In particular, heart failure is the final stage of a series of diseases such as arterial hypertension, ischemic cardiopathy, valve failure, viral myocarditis or the presence of mutations in encoding genes for proteins involved in the sarcomer structure. After the considerable improvement brought about by new diagnostic and therapeutic approaches in managing ischemic cardiopathy, heart failure is the greatest problem for cardiovascular medicine, and as life expectancy increases, hear failure is forecast to be the main cause of disability from 2020 onwards. The therapeutic strategy for hear failure is still based on the use of drugs that tackle the problem mostly by hampering haemodynamic mechanisms. There is no therapeutic approach directed at the heart muscle to try to support adaptive molecular mechanisms, under conditions of increased haemodynamic overload.

Some non transgenic animal models have been generated by surgical approach or by infusion of vasoactive substances (coarctation of the thoracic aorta, chronic infusion with Angiotensin II, etc.), which mimic the condition of arterial hypertension with the development of cardiac hypertrophy, but there is a lack of adequate genetic models. The most common genetic model currently used for this purpose (the SHR rat)¹⁹ develops cardiomyopathy only at the age of 18 months, and this is an obstacle both from the methodological viewpoint (need for longer times) and from the scientific viewpoint (influence of age-related factors). Moreover, the rat is not a suitable model to in vivo characterise the molecular paradigm that sustains phenotypic alteration to be applied for humans. It is interesting to note that in the plethora of transgenic murine models that exhibit elevated levels of arterial pressure (e.g. genic ablation of eNOS, or of IGF-1, or of fibulin 5)²⁰⁻²¹ it is not common to find a transgenic murine model in which an association is noted between the increase in pressure values and an unfavourable left ventricle remodelling of dilated cardiomyopathy with signs of hear failure.

The mouse of the present invention, homozygote with K-ras ^{(Hras)} genotype develops this cardiac pathology already at the age of 2 months, indicating that arterial hypertension must find a favourable condition able to lower the threshold for the development of dilated cardiomyopathy in conditions of increased cardiac overload.

Therefore, the object of the present invention is a non human transgenic animal of K-ras ^{(Hras)/(Hras)} genotype **characterized in that the two endogenous K-ras alleles have been replaced with H-ras alleles**. Preferably, the animal belongs to the murine genus, more preferably it belongs to the Mus musculus species.

A further object of the invention is a method for assaying the activity of a substance comprising the administration of said substance to the non human transgenic animal of the invention, in effective quantities and modes **wherein said activity is cardiotherapeutic activity or anti-hypertensive activity**. Preferably, the substance has an activity on dilated cardiomyopathy.

A further object of the invention is a method for assaying the activity of a substance comprising the **administration of** said substance in appropriate quantities and times to cardiomyocytes, smooth muscular or endothelial vascular cells derived from the non human transgenic animal according the invention **wherein said activity is cardiotherapeutic activity or anti-hypertensive activity.**

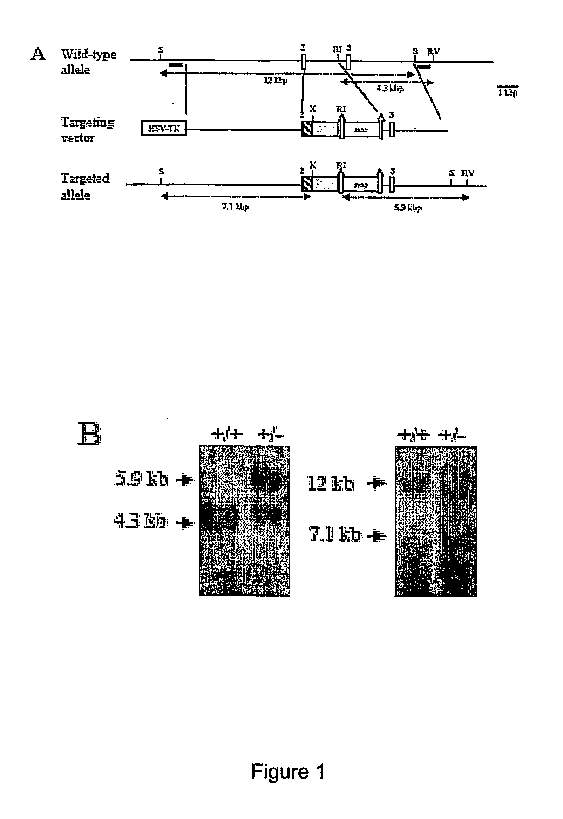

The present invention will now be described in illustrative but non limiting examples thereof, with reference to the following figures and tables: Figure 1. Targeting strategy of knock-in of H-ras (or K-ras 4B) cDNA in K-ras locus.
(A) Wild-type-type K-ras targeted region with targeting vector and the predicted mutant locus. The coding exons are represented by open boxes. The HSV-TK (herpes simplex virus thymidine kinase) and neo cassettes are indicated. The cloned coding sequences for H- or K-ras 4B are represented by dashed boxes. The 3' untranslated region is represented by a grey box. The probes used for Southern hybridization are shown by black boxes and the expected sizes of the signals are indicated by dashed lines. EI, EcoRI; EV, EcoRV; S, SacI; X, Xhol. (B) Southern blot analysis of genomic DNA from ES clones using 3' (left) and 5' (right) probes. +/+, wild-type type; +/-, HrasKI heterozygous.

Fig 2. Expression of the Ras isoforms in different tissues from wild-type-type (WT), KrasKI and HrasKI adult mice.

The protein extracts were probed with antibodies specific for each Ras protein. Pan-Ras antibody was used to detect the total expression levels of Ras proteins. Tubulin antibody was used to normalize the amount of loaded proteins.

Fig 3. Left ventricular remodelling.
(A) Representative M-mode left ventricular echocardiographic recordings of wild-type-type (+/+), KrasKI and HrasKI mice. All measurements were determined in a short-axis view at the level of papillary muscles. (B) Collagen deposition (light blue), indicative of fibrosis, evaluated by Masson's trichrome staining in the left ventricle. (C) Left ventricles processed for histology to measure the cardiomyocyte cross-sectional area from +/+, KrasKI and HrasKI mice. (D) Mean values from 200 cardiomyocyte area determinations per mouse. *P<0.05 versus +/+.

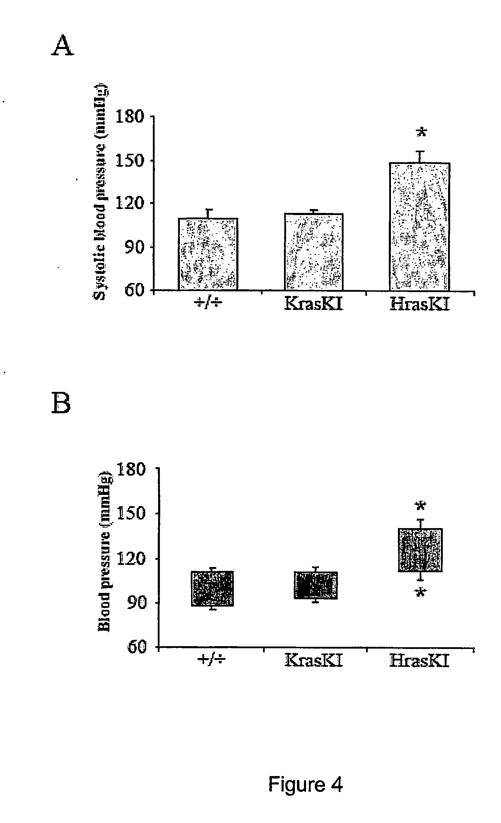

Fig 4. Blood pressure (BP) measurements.
(A) Systolic BP evaluated non-invasively by tailcuff measurements in conscious mice (n = 6 for each group). *P<0.01 versus wild-type type (+/+) and KrasKI. Data are mean ± s.e.m. (B) Systolic and diastolic BP evaluated invasively by radiotelemetry in conscious mice (n = 3 for each group). *P< 0.02 versus +/+ and KrasKI. Data are mean ± s.e.m.

**Table 1. Quantification of genotypes of the progenies from crossings between H-rasKI mice**

| | **wt** | **HrasKI heterozygote** | **HrasKI homozygote** |
|---|---|---|---|
| Total no. of animals (C57/BL6:129/Sv) | 20 | 42 | 19 |
| Total no. of animals (129/Sv) | 14 | 28 | 16 |

| | | | |
|---|---|---|---|
| The statistical χ² obtained supports a Mendelian type of heredity. | | | |

**Table 2. Echocardiographic parameters in basal conditions of wild-type and HrasKI or KrasKI homozygote 129/sv mice.**

| | **WT** | **HrasKI** | **KrasKI** |
|---|---|---|---|
| | n=3 | n=6 | n=3 |
| BW, g | 29.9±1.2 | 26.55±1.14 | 25.16±0.91 |
| HR, **bpm** | 586±29 | 568±25 | 570±20 |
| LVEDD, mm | 3.02±0.17 | 4.22±0.18* | 3.09±0.03 |
| LVESD, mm | 1.29±0.09 | 2.77±0.23** | 1.24±0.006 |
| IVSTD, mm | 0.64±0.98 | 0.49±0.025* | 0.67±0.04 |
| PWTD, mm | 0.67±0.025 | 0.46±0.02** | 0.66±0.05 |
| RWT | 0.43±0.04 | 0.22±0.02** | 0.43±0.03 |
| LVMI, **mg/g** | 1.90±0.14 | 2.5±0.08* | 2.4±0.10 |
| FS% | 57.4±0.75 | 35.14±2.8** | 59.7±0.30 |

| | | | |
|---|---|---|---|
| LVEDD = left ventricular end diastolic diameter, LVESD = left ventricular end systolic diameter, IVSTD = interventricular septum end diastole diameter, PWTD = posterior wall thickness in end diastole, FS = percent fraction of shortening, RWT = relative wall thickness, LVMI = left ventricular mass index, BW =body weight, HR= hear rate. *p>0.05, **p>0.01 vs WT and KrasKI (ANOVA) | | | |

### Methods

### Construction of the vector used for homologous recombination

A genomic DNA clone containing K-ras locus was identified from a genomic library PAC 129/Sv (YAC Screening Centre, DIBIT-HSR) by means of PCR analyses. The PCRs with specific primers, the mapping and the partial sequence have demonstrated that all K-ras exons were contained in the clone. About 20 kbp comprising the exon 2, the exon 3 and the exon 4B were subcloned in the pBluescript KS vector (Stratagene). The vector for homologous recombination was constructed in pBluescript KS using the following DNA fragments: a fragment of genomic DNA of 4.9 kbp containing the 3' end of the intron 2, a fragment of 3.1 kbp containing the 3' end of the intron 3, the exon 3 and a 5' end of the intron 4, the gene coding for the resistance to neomycin of 1.7 kbp derived from the pFlox plasmide with an additional loxP site in the EcoR1 site, the gene coding for the thymidine kinase of the Herpes simplex virus (named here TK) derived from the pFlox plasmide. The two expression cassettes for neo and TK were positioned in such a way that one will be transcribed in the same orientation as K-ras and the other one in the opposite orientation. The 3'-UTR of K-ras was amplified by PCR from a genomic subclone. The H-ras coding sequence was amplified by RT-PCR on total RNA from mouse kidney. The fusion between the exon 2 of K-ras and the remaining part of the coding sequence of H-ras was produced by PCR. In short, the 3' of the intron 2 and the 5' of the exon 2 of K-ras were amplified using as primer at 5' the oligonucleotide:
5'-CTTAGTCTCTAGAGGAACTTCTGTTG-3' (SEQ ID. No. 1) and as primer at 3' the oligonucleotide:
5'-TCTTGACCTGCTGTGTCGAG-3' (SEQ ID No. 2).

In a separate PCR reaction, the remaining part of H-ras coding sequence was amplified using a primer at 5' the oligonucleotide 5'-GACACAGCAGGTCAAGAAGAGTAT-3' (SEQ ID No. 3) and a primer at 3' the oligonucleotide: 5'-TATCTCGAGTCAGGACAGCACACATTTGC-3' (SEQ ID No. 4).

This last oligo comprises the recognition site for the Xhol enzyme (in italics). The primer 3' of the first amplification and the primer 5' of the second one have a complementary segment (nucleotides in common between K-ras and H-ras, shown underlined). Therefore, a recombinant product is formed when the superimposition is extended in a successive PCR reaction and this recombinant product is amplified with the inclusion of external primers in the last extension. The final amplification product (fusion between the exon 2 of K-ras and the coding sequence of H-ras) was verified by sequence. The same strategy was used for the fusion between the exon 2 of K-ras and the remaining part of the coding sequence of K-ras 4B, using for the amplification thereof the oligos:
5'-CTCGACACAGCAGGTCAAGA-3'(SEQ ID No. 5);
5'-TATCTCGAGTCACATAACTGTACACCTTGT-3' (SEQ ID No. 6).

### Homologous recombination and generation of mutant mice

The ES RI cells were cultivated essentially as previously described (Gene Targeting; A practical approach. Edited by A.L. Joyner). 100 µg of the linearised vector were transfected in 2x10⁷ ES cells through electroporation (240 V, 500µF; Gene pulser; Bio-Rad). The ES cell clones were isolated from a culture with G418 (GIBCO BRL) and Ganciclovir, expanded and analysed by Southern blot as shown in Fig. 1. The chimeric mice were generated by microinjection of the transfected ES cells into the blastocysts of C57/BL6 mice, then transferred into pseudo-pregnant females for further development, essentially as described previously. The male chimeric mice were crossed with C57/BL6 or 129/Sv females to generate lines having a mixed or pure genetic strain background, and the transmission of germinal line of the embryonic stem cell markers was identified by the agouti coloration of the fur. The genotype of the progeny was analysed on tail DNA by Southern blot analysis as shown in Fig. 1.

### Western blot Analysis

Different tissues were drawn from 4 week old mice of the three different genotypes (wild-type, KrasKI, HrasKI). They were immediately homogenised in a lysis buffer (50 mM Tris-HCl pH 8.0, 150 Mm NaCl, 5 mM MgCl₂, 1% Triton X-100, 0.5% deoxycholate sodium, 0.1% SDS, 0.5 mM PMSF and a cocktail of protease inhibitors (Sigma)). The lysates were incubated for 30 minutes at 4°C in agitation on wheel and centrifuged, the cellular debris was discarded and the supernatant was collected. 50 µg of proteins from the different tissues of each genotype were separated by electrophoresis on 4-15% SDS-polyacrylamide gradient and then transferred on an Immobilon membrane (Millipore). The immunoblots were probed with monoclonal antibodies anti-K-ras (F234), anti-N-ras (F155) and anti-H-ras (F235), and with anti-pan ras and anti-tubulin antibodies (Santa Cruz Biotechnology, Inc.), and visualised by chemiluminescence (ECL, Amersham) according to the manufacturer's protocol.

The antibodies were detached from the filters incubating them in 62.5mM Tris-Cl (pH 6.8), 0.7% 2-mercaptoethanol and 2% SDS for 30 minutes at 55°C.

### Histological and immunohistochemical analysis

Embryos at different gestation periods were dissected from the mother tissue and from the vitelline sack. The vitelline sack was processed for genotype analysis by Southern blot. The embryos were fixed in 4% paraformaldehyde, dehydrated with ethanol passages, passed in xylene, included in paraffin and sectioned. Serial sections of 7 µm were stained with hematoxylin and eosin for the histological exam.

Apoptosis was monitored on sections included in paraffin using the Cleaved

Caspase-3 (Asp 175) antibody (Cell Signaling Technology) according to manufacturer's instructions.

### Trans-thoracic Echocardiography

Echocardiographic analysis was conducted using a commercially available echocardiograph (System Five Performance, General Electric Vingmed, Waukesha, Wisconsin) equipped with an image transducer at10MHz. Two-dimensional traces were recorded in M-mode from the short axis of the left ventricle, at the papillary muscle level. The echocardiographic images were analysed with NIH-Image program. The thickness of the posterior wall and of the interventricular septum at diastole end, and the diameter of the left ventricle at systole end and at diastole end were measured from at least 3 consecutive cardiac cycles. Ventricular mass was calculated using the Deveraux formula (MV = 1,055 * (VSd + PP + SIV)³ - VSd³, where VSd is ventricle diameter at diastole end, PP the rear wall and SIV the interventricular septum). The shortening fraction was calculated as FA = [(VSd - VSs) / VSd * 100], and the relative wall thickness was calculated as RWT = 2 * PP / VSd.

### Measurement of arterial pressure by telemetry

The mice were anesthetised, and an inguinal incision was performed exposing the femoral artery. The artery was cannulated with a catheter for the transmission of arterial pressure connected to a sensor situated in a commercially available device (Data Sciences International). Such device, with a volume of 2cc, was applied subcutaneously in the animal's back and anchored to the dorsal muscles. After surgical implant of the radiotelemetric device,mice were housed in single cages to recover from anesthesia. Systolic and diastolic arterial pressure and cardiac frequency were recorded in conscious mice, freely moving in the cage, and without any contact with the experimenter. Pressure and heart rate signals were captured with a receiver positioned under each cage and transmitted to a computerised data acquisition and analysis system.

### Results

### Generation of knock-in mice

The locus K-ras extends for about 30 kbp. The genic structure of K-ras consists of 5 exons, with a non coding exon at 5' (exon 0) and two alternative exons (4A and 4B) coding for the C-terminal region (Fig. 1A). The aminoacids coded by the exon 1 and by most of the exon 2, comprising the effector domain, are identical in mammal K-ras and H-ras. The vector for homologous recombination was devised inserting the coding region of H-ras under the control of sequences of K-ras and in such a way as to minimally modify the genomic structure of the locus. Therefore, a part of the coding sequence of H-ras followed by the 3'UTR region of K-ras was merged according to the reading code with the exon 2 of K-ras, and, as a result of the homologous recombination, this cassette was inserted between the exon 2 and the exon 3 of K-ras. Thus, the modified gene codes for H-ras protein instead of K-ras (Fig. 1A). A similar strategy was used to produce the vector for the knock-in of K-ras in K-ras.

After introduction of the vector for homologous recombination into the ES 129/Sv cells, the resulting clones, resistant to G-418 and Ganciclovir, were analysed by Southern blot of genomic DNA digested with EcoRI and Eco RV using a probe located at the 3' of the sequence present in the vector for homologous recombination, at the opposite end from the TK cassette. This probe reveals an EcoRI-EcoRV fragment of 4.3 kbp from the wild-type DNA and a specific additional mutant fragment EcoRI-EcoRV of 5.9 kbp (Fig. 1B). 5 of the 120 clones of ES cells probed for the knock-in of H-ras in the K-ras locus and 4 of the 85 clones of ES cells probed for the knock-in of K-ras in the K-ras locus acquired the exogenous sequences by homologous recombination, as revealed by the probe 3', and therefore they are heterozygous for the mutation (H-rasKI or H-rasKI ). The positive clones for homologous recombination at 3' were further analysed by Southern blot of genomic DNA digested with SacI and Xhol, using an external probe at 5' (Fig. 1B).

### K-ras is not an essential gene for development

The chimeric animals were produced injecting ES H-rasKI cells in C57/BL6 embryos at the blastocyst stage, and these were crossed with C57/BL6 mice to determine the contribution of the germinal line. Four clones of H-rasKI heterozygote ES cells and three clones of KrasKI heterozygote ES cells, tested, produced chimeras that transmit the K-ras mutant allele through the germinal line as determined by Southern blot analysis. No phenotype was observed in heterozygote mutant mice, which grow normally and are fertile.

To determine whether H-ras can replace the functions of K-ras during mouse development, HrasKI heterozygous mice were crossed between themselves and the genotype of the resulting progeny was determined. Since the effect of the mutations could vary depending on the mouse genetic background, the knock-in was performed on pure 129/Sv and mixed C57/BL6:129/Sv strains. Analysis of the genotype of the progeny revealed the presence of homozygous HrasKI animals, indicating that K-ras is not an essential gene for mouse embryogenesis. For each type of crossing, the expected genotypes were present in Mendelian ratio both for the pure and the mixed strain backgrounds, indicating that HrasKI homozygous mice develop normally (Table 1). The ratios between male and female HrasKI progeny were as expected as well. The mice appeared morphologically normal and indistinguishable from the wild-type progeny and were found to be fertile. The weight gain rate of homozygous mutant mice was comparable to that of the wild-type mice. The oldest HrasKI mice now are 8 months old and they are healthy.

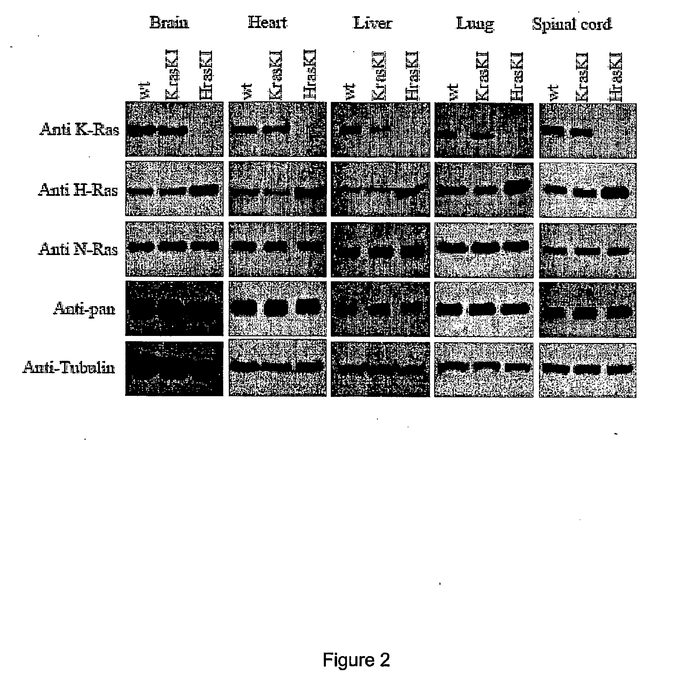

To confirm that K-ras locus had actually been rearranged, in such a way as to prevent the synthesis of any K-ras genic product, and that additional copies of the H-ras gene are expressed by the same locus, an expression analysis was conducted by Western blot on different adult tissues (brain, heart, liver, lung, spinal marrow) from wild-type, Kras4BKI and HrasKI mice (Fig. 2). The lysates obtained from the aforesaid tissues were probed with specific antibodies for K-ras or H-ras showing that the K-ras protein is not present in homozygous HrasKI mice. By contrast, H-ras levels are much higher in these mice compared to wild-type and KrasKI mice, as expected (Fig. 2). To verify whether the N-ras level or the total quantity of Ras proteins was altered, the different extracts were probed also with anti-N-ras and anti-pan-ras antibodies: no significant change in the level of N-ras proteins and of total ras was observed in the tissues of knock-in mice with respect to wild-type mice. These experiments, moreover, allowed to verify that KrasKI mice show completly similar levels of expression of the three ras proteins to those in wild-type mice. This indicates that no essential regions for the expression of K-ras were altered by the genetic manipulation.

### Adult HrasKI mice suffer from dilated cardiomyopathy and arterial hypertension.

It has been shown that the hearts of K-ras knock-out embryos exhibit thinner ventricular walls than those of wild-type mice, at day 15.5 of gestation¹⁵. These data suggest a role of K-ras in heart development. To verify whether, the replacement of both copies of K-ras by H-ras causes heart defects, cardiac morphology was analyzed in embryos and adults of homozygous HrasKI mice, and cardiac functionality studies were performed on adult mice.

The morphology of KrasKI and HrasKI embryo hearts is similar to that of the wild-type mice, at least until day 16.5 of gestation (data not shown).

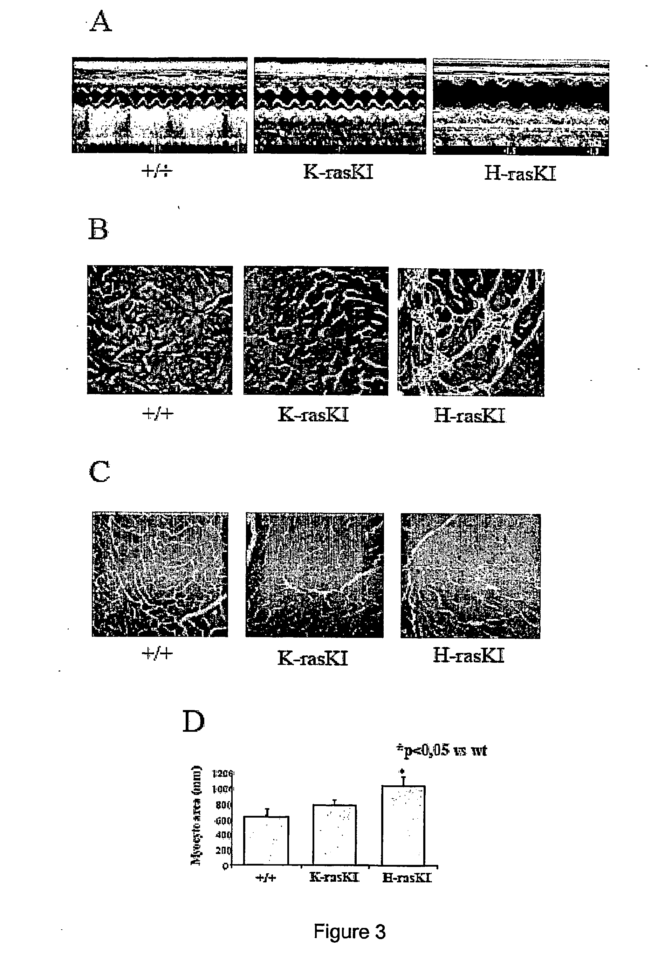

The morphology and the basal cardiac function were examined by transthoracic echocardiography in awake 10-12 week old mice (Fig. 3A). The HrasKI mice show a marked increase in diameter and a significant reduction in the thickness of the septum and of the posterior wall of the left ventricular end diastolic diameter if compared to those of wild-type or KrasKI mice. Therefore, HrasKI mice show the typical picture of dilated cardiomyopathy associated to heart failure (Table 2). The result of the echography are fully supported by histological analysis, which shows both an increase in the cardiomyocytic area and a fibrosis in the left ventricles of H-rasKI mice (Fig. 3B,C,D). It is important to note that KrasKI mice show no difference with respect to wild-type mice, demonstrating yet again that the manipulation of the K-ras locus determines no significant phenotype in itself, and that the Kras4A isoform is not essential for the examined functions (Fig. 3).

To verify whether the dilation and the cardiac dysfunction of HrasKI is associated with conditions of altered cardiac load, the arterial pressure was measured in 10-12 week old HrasKI mice both by non invasive measurement from the tail and invasive measurement by means of a radiotelemetric catheter implanted chronically in the femoral artery. These measurements were made in awake mice, freely moving in the cage, and without any contact with the experimenter. Interestingly, the results obtained with both methods have revealed that HrasKI mice exhibit an increase in systolic pressure (Fig. 4A) and both systolic and diastolic arterial pressure values (Fig. 4B) with respect to KrasKI and wild-type mice, suggesting that the dilated cardiomyopathy observed in HrasKI mice may be secondary to a long-lasting hypertensive condition.

Although different researches have suggested a putative role of Ras proteins in hypertrophic remodelling both *in vivo* and *in vitro*^{22,23,24}, this is the first evidence that correlates Ras with arterial pressure homeostasis control. Additional experiments are ongoing to further investigate the mechanisms that cause the development of hypertension in HrasKI mice.

In conclusion, the results of the authors of the present invention demonstrate that there is no specific need for the KRas protein during mouse development, since the expression of H-ras, guided by the regulatory sequences of the K-ras gene is able to rescue the wild-type embryo phenotype. In adult life, instead, K-ras function is not fully performed by H-ras, since HrasKI mice develop hypertension and dilated cardiomyopathy, thereby revealing a new function of K-ras in cardiovascular homeostasis.

### Bibliography

1.Malumbres, M. & Barbacid, M. Nat Rev Cancer 3, 459-65 (2003).
2.Bar-Sagi, D. Mol Cell Biol 21, 1441-3 (2001).
3.Shields, J.M., Pruitt, K., McFall, A., Shaub, A. & Der, C.J. Trends Cell Biol 10, 147-54 (2000).
4. Downward, J. Nat Rev Cancer 3, 11-22 (2003).
5.Leon, J., Guerrero, I. & Pellicer, A. Mol Cell Biol 7, 1535-40 (1987).
6.Ise, K. et al. Oncogene 19, 2951-6 (2000).
7.Umanoff, H., Edelmann, W., Pellicer, A. & Kucherlapati, R. Proc Natl Acad Sci U S A 92, 1709-13 (1995).
8.Esteban, L.M. et al. Mol Cell Biol 21, 1444-52 (2001).
9.Plowman, S.J. et al. Mol Cell Biol 23, 9245-50 (2003).
10.Pells, S. et al. Oncogene 15, 1781-6 (1997).
11.Wang, Y. & You, M. Exp Lung Res 27, 255-67 (2001).
12.Magee, T. & Marshall, C. Cell 98, 9-12 (1999).
13.Hancock, J.F. Nat Rev Mol Cell Biol 4, 373-84 (2003).
14.Johnson, L. et al. Genes Dev 11, 2468-81 (1997).
15.Koera, K. et al. Oncogene 15, 1151-9 (1997).
16.Ross, J., Jr. Circ J 66, 219-24 (2002).
17.Hoshijima, M. & Chien, K.R. J Clin Invest 109, 849-55 (2002).
18.Chien, K.R., Ross, J., Jr. & Hoshijima, M. Nat Med 9, 508-9 (2003).
19.Boluyt MO, Bing OH, Lakatta EG. Eur Heart J. 16 Suppl N:19-30 (1995)
20.Yanagisawa H, Davis EC, Starcher BC, Ouchi T, Yanagisawa M, Richardson JA, Olson EN. Nature 415(6868):168-171 (2002)
21.Nakamura T, Lozano PR, Ikeda Y, Iwanaga Y, Hinek A, Minamisawa S, Cheng CF, Kobuke K, Dalton N, Takada Y, Tashiro K, Ross Jr J, Honjo T, Chien KR. Nature 415(6868):171-5 (2002)
22.Sugden, P.H. & Clerk, A. Trends Cardiovasc Med 10, 1-8 (2000).
23.Thorburn, A. et al. J Biol Chem 268, 2244-9 (1993).
24.Bergo, M.O. et al. J Biol Chem 279, 4729-36 (2004).

## Claims

1. A non-human transgenic mammalian animal of K-ras ^{(Hras)/(Hras)} genotype, **characterized in that** the two endogenous K-ras alleles have been replaced with H-ras alleles.

2. The transgenic animal according to claim 1, wherein the animal belongs to a murine genus.

3. The transgenic animal according to claim 2, wherein the animal belongs to the Mus musculus species.

4. A method for assaying the activity of a substance, wherein said substance has been administered to the non-human transgenic animals of any of claims 1 to 3, and wherein said activity is cardiotherapeutic activity or anti-hypertensive activity.

5. The method of claim 4, wherein the cardiotherapeutic activity is on dilated cardiomyopathy.

6. A method for assaying the activity of a substance, comprising the administration of said substance to cardiomyocytes, smooth muscle or endothelial vascular cells derived from the transgenic animals of any of claims 1 to 3, wherein said activity is a cardiotherapeutic activity or anti-hypertensive activity.

## Patentansprüche

1. Nicht-humanes, transgenes Säugetier vom K-ras^{(Hras)/(Hras)}-Genotyp, **dadurch gekennzeichnet, dass** die zwei endogenen K-ras-Allele durch H-ras-Allele ersetzt wurden.

2. Transgenes Tier nach Anspruch 1, wobei das Tier zu einer murinen Gattung gehört.

3. Transgenes Tier nach Anspruch 2, wobei das Tier zu der Mus musculus Spezies gehört.

4. Verfahren zum Testen der Aktivität einer Substanz, wobei die Substanz an die nicht-humanen transgenen Tiere nach einem der Ansprüche 1 bis 3 verabreicht wurde, und wobei die Aktivität eine kardiotherapeutische Aktivität oder eine anti-hypertensive Aktivität ist.

5. Verfahren nach Anspruch 4, wobei die kardiotherapeutische Aktivität auf dilatierte Kardiomyopathie wirkt.

6. Verfahren zum Testen der Aktivität einer Substanz, umfassend das Verabreichen der Substanz an Kardiomyozyten, glatte Muskel- oder endotheliale vaskuläre Zellen, die von den transgenen Tieren nach einem der Ansprüche 1 bis 3 abgeleitet sind, wobei die Aktivität eine kardiotherapeutische Aktivität oder eine anti-hypertensive Aktivität ist.

## Revendications

1. Animal mammifère transgénique non humain de génotype K-ras^{(Hras)/(Hras)}, **caractérisé en ce que** les deux allèles K-ras endogènes ont été remplacés par des allèles H-ras.

2. Animal transgénique selon la revendication 1, l'animal appartenant à un genre murin.

3. Animal transgénique selon la revendication 2, l'animal appartenant à l'espèce Mus musculus.

4. Procédé de test de l'activité d'une substance, ladite substance ayant été administrée aux animaux transgéniques non humains de l'une quelconque des revendications 1 à 3, et ladite activité étant l'activité cardiothérapeutique ou l'activité antihypertensive.

5. Procédé selon la revendication 4, dans lequel l'activité cardiothérapeutique est sur une cardiomyopathie dilatée.

6. Procédé de test de l'activité d'une substance, qui comprend l'administration de ladite substance aux cardiomyocytes, aux muscles lisses ou aux cellules vasculaires endothéliales dérivées des animaux transgéniques selon l'une quelconque des revendications 1 à 3, ladite activité étant une activité cardiothérapeutique ou antihypertensive.


### SEQUENCE LISTING

<110> BIOGEM S.c.ar.l
   Potenza, Nicoletta
   Di Lauro, Roberto
   De Vita, Gabriella
   Lembo, Giuseppe
<120> Animal model for studying substances with cardiotherapeutic and
   anti-hypertensive activity
<130> PCT89843
<150> RM2004A000569
   <151> 2004-11-18
<160> 6
<170> PatentIn version 3.3
<210> 1
   <211> 26
   <212> DNA
   <213> Artificial
<220>
   <223> fusion Kras-Hras primer 5'
<400> 1
   cttagtctct agaggaactt ctgttg 26
<210> 2
   <211> 20
   <212> DNA
   <213> Artificial
<220>
   <223> Fusion Kras-Hras primer 3'
<400> 2
   tcttgacctg ctgtgtcgag 20
<210> 3
   <211> 24
   <212> DNA
   <213> Artificial
<220>
   <223> remaining Hras part primer 5'
<400> 3
   gacacagcag gtcaagaaga gtat 24
<210> 4
   <211> 29
   <212> DNA
   <213> Artificial
<220>
   <223> remaining Hras part primer 3'
<400> 4
   tatctcgagt caggacagca cacatttgc 29
<210> 5
   <211> 20
   <212> DNA
   <213> Artificial
<220>
   <223> fusion Kras-Kras primer 5'
<400> 5
   ctcgacacag caggtcaaga 20
<210> 6
   <211> 30
   <212> DNA
   <213> Artificial
<220>
   <223> fusion Kras-Kras primer 3'
<400> 6
   tatctcgagt cacataactg tacaccttgt 30